# EUROPEAN PATENT APPLICATION

(11) **EP 2 425 826 A1**
(43) Date of publication of application: **07.03.2012**
(21) Application number: 10174940.6
(22) Date of filing: 01.09.2010
(51) Int. Cl.: A61K 9/28, A61K 31/60

(54) **Mesalazine tablet having improved dissolution**

(71) Applicant: Disphar International B.V., 7255 PZ Hengelo (NL)
(72) Inventor: The designation of the inventor has not yet been filed
(74) Representative: Pochart, François

(57) **Abstract**

The invention provides a method for preparing a mesalazine enteric coated tablet comprising: (i) granulating a composition comprising mesalazine, a pharmaceutically acceptable salt, or ester thereof, into mesalazine granulates; (ii) tabletting a core composition comprising the mesalazine granulates obtained in (i) to obtain a tablet core; (iii) coating the tablet core obtained in (ii) with at least an enteric coating; where the tablet core hardness is controlled to be comprised between 80 N and 105 N.

## Description

### BACKGROUND OF THE INVENTION

Mesalazine or mesalamine is an aminosalicylate that is prescribed for the treatment of Inflammatory Bowel Disease (IBD). IBD can manifest itself in a variety of forms, the most common of which are Crohn's disease and ulcerative colitis (UC). Crohn's disease (CD) is a chronic transmural inflammation of the bowel which can affect the whole gastrointestinal tract, usually in a discontinuous pattern. The initial location of CD is most commonly in the lower ileum. From here the inflammation typically spreads towards proximal parts of the small intestine. However, the colon is also often involved. Ulcerative colitis is a chronic inflammatory bowel disease affecting only the colon and shows a continuous distribution in the gastrointestinal mucosa. In most patients mainly the distal part of the colon and the rectum are inflamed with often a proximal spread. In the most severe cases, the whole colon is affected ("pancolitis").

To date it is not possible to cure Crohn's disease or ulcerative colitis. Mesalazine plays an important role in the treatment of both diseases by inducing and maintaining remission in chronic inflammatory bowel diseases. Its main principle of action is a topical effect at the inflamed mucosa. Systemic absorption should be minimized, as this leads to unwanted systemic side-effects and inefficient redistribution of the mesalazine to the sites of inflammation. Therefore, oral mesalazine dosage forms should release the active substance selectively at the inflamed areas in the gastrointestinal tract. Because of the different disease patterns of Crohn's disease and ulcerative colitis, different formulations are required to adequately treat different patient subgroups.

Salofalk® is a known enteric-coated tablet, available on the market. It is comprised of a core with an enteric coating, where the enteric coating is a polyacrylic ether. Salofalk® is a tablet comprised of mesalazine, sodium carbonate, glycine, povidone, microcrystalline cellulose (E460), colloidal anhydrous silica, calcium stearate, hydroxypropylmethyl cellulose (E464), methacrylic acid copolymer, talc, titanium dioxide (E171), iron oxide (E172), polyethylene glycol and polymethacrylate. According to the Summary of Product Characteristics, the main site of action of Salofalk® is said to be the terminal ileum and the ascending colon.

Mesalazine 500 mg EC tablets are available from Disphar. These tablets have the same indication as Salofalk®, i.e. treatment of ulcerative colitis and Crohn's disease. The tablets have been found not to be bioequivalent to Salofalk®. Scintigraphic studies were carried out to establish that the active ingredient is indeed delivered in the ileocaecal region and the ascending colon (see Brunner et al, Aliment Pharmacol Ther 23, 137-144). Due to the presence of an excipient creating a basic environment, it is believed that the mesalazine from the Salofalk® tablets is absorbed somehow more than the mesalazine from the Mesalazine 500 mg EC tablets. Yet these Disphar tablets, since not bioequivalent, have not been shown to be non-inferior to Salofalk®; in other words, the two products have not been demonstrated to be equally effective.

From in vitro dissolution testing, it further appears that currently marketed enteric coated tablets show some variation in release characteristics; dissolution can vary within broad limits.

Hence, there is a need for a novel tablet that would solve the above problems.

### BRIEF SUMMARY OF THE INVENTION

It is an object of the present invention to provide a process for making tablets, wherein the hardness of the tablet core is controlled to be within specified limits, namely between 80 to 105 N, preferably 83 and 103 N, more preferably 88 N to 9 8 N, more preferably 90 to 96 N, especially about 92 to 94 N, whereby dissolution is obtained with a low variability and whereby the resulting EC tablet is equally effective to Salofalk® despite not being bioequivalent. This finding is surprising, since it is generally accepted that generics require bioequivalence. The applicant has shown that a proper selection of hardness, duly controlled, will produce tablets with a specified dissolution and which will be non-inferior to Salofalk®. The hardness has been shown to be related to the dissolution rate of the tablet. Dissolution rate may not be directly related to hardness; rather it is related through the disintegration. According to L. Lachman et al (L. Lachman, H.A Lieberman, J.L Kanig, The theory and practice of industrial pharmacy. 1976. Second Edition pp 112-116. Lea & Febiger) the disintegration time of tablets is directly proportional to the compression force and to the tablet hardness. Further they indicate that a high compression force can decrease the dissolution rate of the disintegrated tablet. On the other hand it has been reported according to M.E. Aulton (M.E. Aulton, Pharmaceutics, The science of dosage form design. 2002. Second Edition. pp 411-412. Churchill Livingstone) that a high compression force can in some cases result in a long disintegration time and in other cases result in a short disintegration time. For enteric coated mesalazine tablets J.N.C. Healey (J. N. C. Healey (1990) Gastrointestinal Transit and Release of Mesalazine Tablets in Patients with Inflammatory Bowel Disease, Scandinavian Journal of Gastroenterology, Vol. 25, No. s172, pp 47-51, DOI 10.3109/00365529009091910) has also indicated that there also is a correlation between the disintegration and drug absorption in the human body. What can be concluded from the above references is that dissolution cannot be predicted directly from the hardness of the tablet. The applicant has been able to show this relationship and, in a surprising manner, been able to show that the resulting dissolution could afford a tablet that is non-inferior to Salofalk®.

The tablets of the invention comprise a core and an enteric coating disposed thereon. The invention further provides a method of treating a patient suffering from an inflammatory bowel disease using the tablets thus prepared.

The invention thus provides a method for preparing a mesalazine enteric coated tablet comprising:
(i) granulating a composition comprising mesalazine, a pharmaceutically acceptable salt, or ester thereof, into mesalazine granulates;
(ii) tabletting a core composition comprising the mesalazine granulates obtained in (i) to obtain a tablet core;
(iii) coating the tablet core obtained in (ii) with at least an enteric coating
where the tablet core hardness is controlled to be comprised between 80 N and 105 N.

According to one embodiment, the final core hardness is controlled to be comprised between about 83 N and 103 N, preferably between 88 N and 98 N, more preferably between about 90 and about 96 N, especially about 92-94 N.

According to another embodiment, the tablet releases not less than about 80% mesalazine after 40 minutes when measured using USP dissolution apparatus II (paddle method), 100 rpm, pH 7.2 phosphate buffer, 37°C.

According to another embodiment, the tablet releases not more than about 10% mesalazine after 15 minutes when measured using USP dissolution apparatus II (paddle method), 100 rpm, pH 7.2 phosphate buffer, 37°C.

According to another embodiment, the process comprises a pre-compression step.

According to another embodiment, the tablet comprises 500 mg, 750 mg, 800 mg or 1000 mg mesalazine.

The tablet is notably for the treatment of inflammatory bowel disease (IBD), including Crohn's disease and/or ulcerative colitis.

### DETAILED DESCRIPTION OF EMBODIMENTS OF THE INVENTION

The tablets of the invention are manufactured according to standard practice, except that the hardness of the final core is precisely controlled. The tablets comprise a core with the active ingredient, where the core is manufactured by first granulating a mixture into granules, optionally mixing with extra-granular phase, compressing into tablet cores and coating with an enteric coating that surrounds a coated tablet core and provides delayed release mesalazine tablets. Thus, mesalazine is preferably released in the ileocaecal region and the ascending colon of the patient, especially the latter. The tablets can be designed to release mesalazine starting at pH 6.5 and to continue to release at higher pHs, for example, at pH 7.0.

The tablet of the present invention thus includes a tablet core and at least one coating layer. Optionally, other coatings can be present, such as a coating providing an appealing appearance enhancing patient's compliance.

The process for manufacturing the tablets of the invention is as follows.

The manufacturing of the mesalazine EC tablets of the invention is divided in three main steps; Granulation, Compression and Coating. A mesalazine batch is usually divided into sub batches. Each sub batch is granulated separately in a high shear mixer, using known techniques. The granules are sieved and dried. The granules are then compressed into tablet cores. The tablet cores are then coated.

The granulation step is as follows.

In the granulation process as used in the invention, a standard apparatus is used. A chopper is installed on the apparatus, as is know to those skilled in the art. Dry mixing is usually performed with the chopper off, but the chopper can also be activated. Granulation liquid is then added from a suitable supply. Granulation liquid is usually water, but other commonly used granulation liquids can be used as well. The granulation liquid can be added simply by pouring it from a reservoir, or it can be sprayed onto the mixture. Liquid addition is also typically carried out with the chopper off, but the chopper can also be activated. Last, there is a granulation step per se, so as to obtain granules. While having the chopper off is possible, the preferred embodiment is with the chopper on. Less water would be used, especially if the water was added by spraying it onto the mixture. Also, longer granulation time would be possible.

The granules are then sieved to minimize variations. A sieve of varying hole diameter (e.g. from 1 to 3 mm, preferably 1.5 to 2.5 mm) can be used. Preferably, the particle fraction >850 µm represents a small part of the particles. In the preferred embodiment, larger particles are avoided so as to improve dissolution.

The granules are then dried. Drying cabinets can be used, albeit this is not the preferred process. A fluid bed dryer (FBD) can be used for drying the granules. Vacuum drying is also possible. FBD is preferred. Low and constant Relative Humidity is preferred during the drying step. Constant temperature (of both the inlet air and the granules) is also preferred during the drying step. The drying can be controlled by monitoring the LoD. Tight specifications will enhance dissolution; typically the LoD is 0.5-2.5% on the granules. It is further possible to use the product temperature as an end point value for the drying, instead of the LoD.

As the granule quality may affect the tabletting properties, it is preferred that a consistent granule quality be obtained, so as to allow tight final tablet properties.

For example, the particle size distribution of the granules is such that d(0.1) is from 6 to 20 µm, the d(0.5) is from 100 to 350 µm and the d(0.9) is from 500 to 1000 µm, preferably d(0.1) is from 8 to 15 µm, the d(0.5) is from 150 to 280 µm and the d(0.9) is from 580 to 830 µm.

The tabletting process is as follows.

The resulting granules are then tabletted, after an extra-granular phase is added in a mixer (e.g. a tumble mixer). Tabletting is carried out with standard equipment. Any punch shape can be used, and suitable shapes of the tablet core include round, oval shaped, conical, capsule shaped, and biconical; a preferred shape is oblong, preferably with a high tablet waist.

The specifications for the tablet core hardness are tight and the hardness is selected such that the tablet core (i.e. without any coating on it) has a hardness of 80 to 105 N, preferably 83 to 103 N, more preferably 90 to 96 N, especially about 92-94 N. It has been found that the precise and tight tablet core hardness allows obtaining a defined dissolution and allows for the final EC tablet to be as effective as Salofalk®, despite lack of bioequivalence. Such a result is surprising, since usually bioequivalence is the driving factor for ensuring two different drug formulations to have substantially the same effect. Further, WO-A-2009/090484 indicates that the amount of coating applied may provide different dissolution profiles. The Applicant has found that by controlling tightly the hardness of the tablet, the coating will have a minimal impact and the driving factor is the tablet core hardness.

A pre-compression step is preferably used. This pre-compression step is performed in the same apparatus as the compression step. This pre-compression step is a standard technique, usually carried out with a pressure different from the compression pressure.

Process control comprises different measures. The first measure that is carried out on each batch is the measurement of the tablet core hardness. If the hardness is not within the prescribed range, or if one desires to have the 92-94 N value to be further approached, it is possible to adjust the process parameters. The usual parameter that is adjusted is the tabletting compression force and/or pre-compression force (if pre-compression is used); however other parameters can be adjusted (such as the granulation parameters, see above). The control of the hardness comprises the measure of the hardness using appropriate tools as is known in the art. Notably, the tools will comply with the necessary quality standards. Tablet hardness testers are known in the art. When a deviation is detected, the process conditions will be modified to result in a change in hardness, so that the target value is reached. Notably the compression forces may be adapted. Hence, it is possible to ensure that the final tablet exhibits the required hardness.

The coating process is as follows.

The tablet cores are then coated. The coating layers can be applied utilizing any suitable method. For example, the coating layers are applied in an automated fluidized bed or a coating pan by spraying the coating composition in a solvent onto the tablet cores. In this process, the aqueous or organic solvent is removed by techniques known to one of ordinary skill in the art, e.g. by drying or during curing. Preferred conditions for the coating include coating temperature such as 35-70°C and atomising pressure such as 1.5-6 bars, as well as precise control of the spray rate. Any suitable concentration of the suspension can be used. The cores are usually coated in three steps. First an isolation coat is applied. The purpose of this coating is to provide a good bonding surface for the second coating layer which is the gastro resistant (enteric) coating. The third layer is a polish that is added to improve the tablet appearance and to facilitate administration of the tablet. When dispersions are used for the coating, the coating dispersion has a solid content, for example, an average solid content, ranging from about 3% to about 20%, preferably from about 4% to about 15%, more preferably from about 5% to about 12% by weight of the dispersion. Suitable solvents include water, ethanol, methanol, isopropyl alcohol, chloroform, acetone, ether, or mixtures thereof. Preferably, the solvent is water or a mixture of ethanol and water.

The tablet core comprises mesalazine, a pharmaceutically acceptable salt or ester thereof, a binder, and at least one intergranular disintegrant. Any suitable amount of the drug can be present in the tablet core, e.g., in an amount about 100 mg or more, 200 mg or more, 300 mg or more; or, in an amount about 1000 mg or less, 900mg or less, 600 mg or less, 500 mg, or less. For example, mesalazine can be present in an amount about 500 mg or about 750 mg or about 800 mg or about 1000mg. Alternatively, mesalazine, salt or ester thereof can be present in an amount about 10% or more, about 20% or more, or about 30% or more, by weight, or about 95% or less, about 85% or less, or about 75% or less by weight of the tablet core. In embodiments, the mesalazine, salt or ester thereof is present in an amount about 30% to about 90%, preferably about 50% to about 80%, and more preferably about 75% to about 77% by weight of the tablet core.

In accordance with the invention, any suitable binder can be employed. The binder holds the components of the tablet core together. Examples of suitable binders include microcrystalline cellulose (which also performs the function of filler as well), povidone, starch, hydroxypropyl cellulose, and mixtures thereof. Other examples of binders include hydroxypropyl methyl cellulose, low-substituted hydroxypropyl ether of cellulose, for example, a hydroxypropyl ether of cellulose having a degree of substitution from 5 to 16 mass % when determined on a dry basis (US Pharmacopoeia, 23rd Ed., pp 2253-2254), glucose, carboxymethylcellulose, dextrin, ethylcellulose, gelatin, pregelatinized starch, and mixtures thereof. Povidone is the preferred binder.

Any suitable amount of binder can be employed to prepare the tablet core. For example, the binder can be present in an amount about 1% or more, 2% or more, about 4% or more, or about 8% or more, or in an amount about 30% or less, about 25% or less, or about 20% or less by weight of the tablet core. In certain embodiments, the binder is present in an amount about 2% to about 10% by weight of the tablet core.

In accordance with the invention, an intergranular disintegrant or superdisintegrant can be present in the tablet core. A disintegrant is a substance in a tablet formulation that enables the tablet to break up in smaller fragments and is generally used at a low level in the solid dosage form, typically 1-10% by weight relative to the total weight of the dosage unit (final coated tablet). Examples of suitable intergranular disintegrants include crospovidone, croscarmellose sodium, and sodium starch glycollate and mixtures thereof. The preferred disintegrant is crospovidone. Any suitable amount of the intergranular disintegrant can be employed. For example, the intergranular disintegrant is present in an amount of about 1% or more, about 4% or more, or about 6% or more, or in an amount about 8% or less, about 5% or less, or about 3% or less by weight of the tablet core. In certain embodiments, the intergranular disintegrant is present in an amount about 1% to about 10%, preferably about 2% to about 7%, and more preferably about 2% to about 5% by weight of the tablet core. Alternatively, the disintegrant can also be used as an intragranular disintegrant, i.e., wherein the disintegrant is used within the mesalazine granulate.

The tablet core can optionally include one or more excipients. These excipients include fillers, glidants, lubricants, and/or wetting agents. Suitable fillers include ethylcellulose and lactose. Suitable glidants include amorphous silica, powdered cellulose, and starch. Suitable wetting agents include sodium dodecyl sulfate (SDS).

Any suitable lubricant can be employed. A lubricant keeps the mixture from sticking to the equipment during the tabletting process. Examples of suitable lubricants include sodium stearyl fumarate (PRUV®), magnesium stearate, colloidal silicon dioxide, and talc. The lubricant can be present in any suitable amount, e.g., in an amount of about 0.1% or more, about 0.5% or more, or about 0.8% or more, or in an amount about 2% or less, about 1% or less, or about 0.5% or less, by weight of the tablet core. In certain embodiments, the lubricant is present in an amount about 0.5% to about 1.5%, and preferably about 0.8% to about 1% by weight of the tablet core.

According to the present invention, the tablet core is then coated, typically with a first, intermediate, coating layer and a second enteric coating layer. It is believed that the first coating increases the adhesion of the second coating layer and/or removes any incompatibility between the tablet core and the second coating layer. The first coating layer is free or substantially free (e.g., less than 1%, 0.5%, or 0.1% by weight of the first coating layer) of a methacrylic acid/methyl methacrylate copolymer. The first coating layer typically comprises a cellulose derivative and/or polyvinylpyrrolidone (or povidone). Any suitable cellulose derivative can be employed, for example, a cellulose ether polymer, preferably a hydrophilic cellulose ether polymer such as hydroxypropyl methylcellulose. Optional additives can be present in the first coating layer, e.g., a polyol such as polyethylene glycol.

The first coating layer can be present in any suitable amount, for example, about 0.1% or more, about 0.5% or more, about 0.8% or more, or in an amount about 2% or less, about 1% or less, or about 0.5% or less, by weight of the tablet. In embodiments, the first coating layer is present in an amount about 0.5% to about 1.5%, and preferably about 0.5% to about 0.9%, by weight of the tablet.

The second coating layer is the enteric coating layer and comprises, based on the coating weight, from 60 to 80% by weight of an acrylic polymer such as a methacrylic acid/methyl methacrylate copolymer. Alternatively, this coating may consist essentially of (e.g., comprises more than 90% by weight especially more than 95% by weight) an acrylic polymer such as a methacrylic acid/methyl methacrylate copolymer.

The enteric coating layer can include additional components, e.g., anti-tack agents, plasticizers and coloring agents such as pigments, lakes, and dyes. Suitable plasticizers include triethyl citrate, diethyl phthalate, triethyl acetyl citrate, triacetin, tributyl citrate, dibutyl phthalate, polyethylene glycol, glycerol, and mixtures thereof. Suitable coloring agents include aluminum lakes, titanium dioxides, iron oxides or natural colors such as riboflavin or carotenoids.

The enteric coating layer can be present in any suitable amount, for example, about 1% or more, about 5% or more, or about 8% or more, or in an amount about 20% or less, about 15% or less, or about 12% or less, by weight of the tablet. The second (enteric) coating layer is present in an amount generally of about 10%, by weight of the tablet.

The first coating layer can be present in an amount of about 0.5% to about 1.5% by weight of the tablet and the second coating layer is present in an amount of about 5% to about 15% by weight of the tablet.

Any suitable acrylic copolymer can be used, such as a methacrylic acid/methyl methacrylate copolymer or an ammonium ethyl acrylate type B copolymer. For example, the acrylic copolymer is available under the trade name EUDRAGIT® (Rohm Pharma GmbH, now Evonik, Germany). A preferred composition is a mixture of Eudragit® 100L/100S.

The enteric acrylic copolymer can be present in any suitable amount, for example, about 1% or more, about 3% or more, or about 5% or more, or in an amount about 20% or less, about 15% or less, or about 12% or less, by weight of the tablet.

An anti-tack agent can be employed in the enteric coating layer. An anti-tack agent is a compound that is used to reduce or minimize tackiness (i.e., stickiness) between coated tablets both during and after the coating process. Talc, glyceryl monostearate, and silica (colloidal) (or silicone dioxide) or mixtures thereof are examples of suitable anti-tack agents. Any suitable amount of the anti-tack agent can be used in the second coating layer.

The enteric coating layer can contain any suitable amount of plasticizer, for example, about 5% or more, about 10% or more, or in an amount about 25% or less, about 20% or less, by weight of the acrylic copolymer. In certain embodiments, the plasticizer is present in an amount about 10% to about 15%, by weight of the acrylic copolymer.

Optionally, a polishing layer may be added on top of the second coating layer. Suitably, the composition of the polishing layer comprises polyethylene glycol.

In accordance with an embodiment of the invention, the tablet releases not less than about 80% mesalazine after 40 minutes when measured using USP dissolution apparatus II (paddle method), 100 rpm (while the USP indicates 50 rpm), pH 7.2 phosphate buffer, 37°C, according to USP reference standard USP32-NF27 or "the USP paddle dissolution test method".

In accordance with an embodiment of the invention, the tablet releases not more than about 10% mesalazine after 15 minutes when measured using USP dissolution apparatus II (paddle method), 100 rpm (while the USP indicates 50 rpm), pH 7.2 phosphate buffer, 37°C, according to USP reference standard USP32-NF27 or "the USP paddle dissolution test method".

The invention thus provides a method for preparing a mesalazine enteric coated tablet having specific hardness values, ensuring an efficacy that is comparable to the one of Salofalk®

The invention also provides tablets and batches of tablets produced in accordance with embodiments of the invention. The present invention further provides a method of treating a patient for an inflammatory bowel disease (IBD) comprising administering to the patient an effective amount of a mesalazine delayed release tablet described above.

The following example further illustrates the invention but, of course, should not be construed as in any way limiting its scope.

### EXAMPLE

This example illustrates a method of preparation of an enteric coated tablet according to an embodiment of the invention.

A wet granulate of mesalazine, microcrystalline cellulose, silicon dioxide and polyvinylpyrrolidone is prepared. After drying, the granules are sieved through a sieve. Subsequently, the sieved granules are blended with microcrystalline cellulose, cross-linked polyvinylpyrrolidone, and magnesium stearate. The resulting blend is compressed to obtain tablet cores. Tablet cores are then coated with an enteric coating comprising a mixture of Eudragit® 100L/100S, talcum, magnesium stearate, triethyl citrate and dyes, with intermediate and finishing coats.

The tablet core hardness is measured so as to have a core hardness between 83 N and 103 N, with a target at 92-94 N.

Batches HA758A, FH772C, GD793A and EE767B are manufactured according to the above process, batches not meeting the requirement for hardness are discarded. The dissolution rates and hardness are given in the table 1 below. The 05G, 06K, 05F and 06E are Salofalk® tablet batches. One can notice a very high variability of the values of time to reach 80% dissolved with the Salofalk® product. In contrast, the invention shows lower values (hence faster dissolution) and less variation.

**Table 1.**

| Batch | Hardness (N) | Time to reach 80% dissolved (min.) |
|---|---|---|
| HA758A | 86.7 | 28.7 |
| FH772C | 83.5 | 36.0 |
| GD793A | 100.2 | 36.1 |
| EE767B | 91.8 | 39.4 |
| 05G18076R | nd | 37.4 |
| 06K24355L | nd | 43.6 |
| 05F02068R | nd | 38.2 |
| 06E021664 | nd | 59.7 |

A non-inferiority study for Ulcerative Colitis has been carried out to determine if the study drug (Mesalazine EC Tablets 500 mg) is at least as effective (non-inferiority testing) as the comparative medication (Salofalk® Tablets 500 mg) in the common dose regimen 3 x 2 Tablets (= 3 g daily). The batch numbers for the tested product according to the invention were FH772C, GD793A and EE767B. The duration of treatment was 8 weeks, with a dose of 3 x 2 Tablets (= 3 g daily). The reference therapy was Salofalk® Tablets 500 mg, same dose.

A randomised, parallel group, comparative efficacy and safety study was carried out.
*Screening:* At screening a complete medical history and evaluation of the major organ systems was conducted. Basic haematology, serum chemistry and urine analysis and pregnancy test was performed and the laboratory assessment for faecal pathogens, UC DAI (Ulcerative Colitis Disease Activity Index) score and colonoscopic disease grading was evaluated within 7 days prior to the baseline visit.
*Visit No. I:* The study drugs were delivered according to the randomisation scheme in the common dose 3 g/day of Mesalazine EC 500 mg or Salofalk® in a double blind regimen, 3 times daily. The UC DAI diary was distributed to patients. If the laboratory assessment for faecal pathogens was found to be positive, the patients were excluded from the trial.
*Visit No. II (week 4* +/- 4 *days):* When the patient did not meet any criteria for premature withdrawal, the UC DAI score on the basis of UC DAI diary was calculated, urine examination, physical global assessment, blood and urine sampling and adverse events were assessed and medication for the next 4 weeks was distributed.
*Visit No. III (week 8* +/- 4 *days):* Same evaluation as visit No. II plus compliance assessment by calculation of returned medication. If the patient was withdrawn before week 8, the visit No. III was registered as the last visit.
Criteria for evaluation:
   *Primary efficacy endpoint:*
      Proportions of patients considered to have achieved clinical remission (UC DAI score < 2 points)
   *Secondary efficacy endpoints:*
      - Change in total UC DAI score (stool frequency, rectal bleeding, endoscopic evaluation, physician's global assessment).
      - Proportions of patients considered to have achieved:
         - partial response: reduction in physicians global assessment + one other component of UC DAI and UC DAI reduction at least of 2 points but the final UC DAI not lower than 3 points
         - no response: no clinical remission, no partial response
Comparison of UC DAI score elements in each patient: stool frequency, rectal bleeding, endoscopic evaluation, physician's global assessment, patient's functional assessment.

### Patient compliance (drug form and dosage)

The study was carried out according to the protocol and strictly followed ICH-GCP guidelines. In total 248 patients were randomised in the study and received double-blind medication. 122 patients were included in the Mesalazine group and 126 patients in the Salofalk® group. The primary variable was reaching UC DAI score of at most 2 at visit 3 (clinical remission). The observed clinical remission rate was 60.5% in the Mesalazine group compared to 56.0% in the Salofalk® group. The treatment difference (Mesalazine - Salofalk®) was 4.5%, the two-sided 95% CI was (-8.1%; 17.1%). The pre-defined non-inferiority margin in the study protocol was -15% and therefore the non-inferiority of Mesalazine could be concluded. No statistically significant treatment differences were detected for following variables: change from baseline in UC DAI, clinical response, rectal bleeding, mucosa, physician's global assessment and patient's functional assessment. These secondary variables were supportive measurements related to the primary objective. The fact that no significant treatment differences could be detected is considered to be supportive evidence for concluding non-inferiority of the test drug.

There were 37 AEs (adverse effects) reported, 14 of them from the Mesalazine group, 23 from the Salofalk® group. There were two AEs reported considered as drug related (one moderate abdominal pain in the Salofalk® group and one mild abdominal pain in the Mesalazine group). No severe AEs were reported.

The analysis revealed that the mesalazine tablets of the invention were non-inferior to Salofalk®, based on primary and secondary endpoints and therefore similar efficacy was demonstrated between both investigated drugs in the treatment of mild to moderate active ulcerative colitis.

A non-inferiority study for Crohn's Disease has been carried out to determine if the study drug (Mesalazine EC Tablets 500 mg) is at least as effective (non-inferiority testing) as the comparative medication (Salofalk® Tablets 500 mg) in the common dose regimen 3 x 3 Tablets (= 4.5 g daily). The batch numbers for the tested product according to the invention were FH772C and HA758A. The duration of treatment was 12 weeks, with a dose of 3 x 3 Tablets (= 4.5 g daily). The reference therapy was Salofalk® Tablets 500 mg, same dose.

A randomised, parallel group, comparative efficacy and safety study was carried out.
Diagnosis and main criteria for inclusion were:
- Established diagnosis of Crohn's disease in Moderate active status verified by clinical evaluation, laboratory tests and by X-ray examination or/and colonoscopy and histology;
- Crohn's disease of at least 3 months duration;
- Acute exacerbation of moderate Crohn's disease with a CDAI (Crohn's Disease Activity Index) score between 220-400 points;
- Patients between 18-75 years, both sexes;

The criteria for evaluation, efficacy, did comprise Remission (primary variable), CDAI, IBDQ (Inflammatory Bowel Disease Questionnaire) (secondary variables).

The remission rate was compared between treatment groups. The observed remission rate was slightly greater for the Mesalazine tablets of the invention (58.1 %) as compared to Salofalk® (55.1 %). Their 95% confidence intervals (CIs) overlapped each other almost entirely. The 95% CI for the treatment difference (Mesalazine - Salofalk®) was from 10.6% to 16.6% and it did not exclude zero, which means that no statistically significant treatment difference could be detected at the *a* = 0.05 significance level. In the study, there is no difference in the adverse effects frequency. Mesalazine EC tablets are declared non-inferior to the reference active comparator Salofalk® tablets. Both treatments were well tolerated and safe.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subj ect matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method for preparing a mesalazine enteric coated tablet comprising:
(i) granulating a composition comprising mesalazine, a pharmaceutically acceptable salt, or ester thereof, into mesalazine granulates;
(ii) tabletting a core composition comprising the mesalazine granulates obtained in (i) to obtain a tablet core;
(iii) coating the tablet core obtained in (ii) with at least an enteric coating where the tablet core hardness is controlled to be comprised between 80 N and 105 N.

2. The process according to claim 1, wherein the tablet core hardness is controlled to be comprised between about 83 N and 103 N, preferably between 88 N and 98 N, more preferably between about 90 N and about 96 N, especially about 92-94 N.

3. The process according to claim 1 or 2, wherein the tablet releases not less than about 80% mesalazine after 40 minutes when measured using USP dissolution apparatus II (paddle method), 100 rpm, pH 7.2 phosphate buffer, 37°C.

4. The process according to any one of claims 1 to 3, wherein the tablet releases not more than about 10% mesalazine after 15 minutes when measured using USP dissolution apparatus II (paddle method), 100 rpm, pH 7.2 phosphate buffer, 37°C.

5. The process according to any one of claims 1 to 4, wherein the process comprises a pre-compression step.

6. The process according to any one of claims 1 to 5, wherein the tablet comprises 500 mg, 750 mg, 800 mg or 1000mg mesalazine.

7. The process according to any one of claims 1 to 6, wherein the tablet is for the treatment of inflammatory bowel disease (IBD).

8. The process according to claim 7, wherein the tablet is for the treatment of Crohn's disease and/or ulcerative colitis.
